# EUROPEAN PATENT APPLICATION

(11) **EP 4 553 155 A1**
(43) Date of publication of application: **14.05.2025**
(21) Application number: 23918665.3
(22) Date of filing: 26.12.2023
(51) Int. Cl.: C12N 5/10, C07K 14/78, C12M 3/00, C12N 5/071, C12N 15/113, C12N 15/12

(54) **METHOD FOR PRODUCING INDUCED PLURIPOTENT STEM CELLS USING EPITHELIAL CELLS**

(30) Priority: 23.01.2023 JP 2023008217; 24.05.2023 JP 2023085614
(71) Applicant: Pola Chemical Industries, Inc., Shizuoka 437-8765 (JP)
(72) Inventor: KUGE, Takayuki, Yokohama-shi, Kanagawa 244-0812 (JP); IWANAGA, Tomoyuki, Yokohama-shi, Kanagawa 244-0812 (JP)
(74) Representative: Witthoff Jaekel Steinecke Patentanwälte PartG mbB
(86) International application number: PCT/JP2023/046566
(87) International publication number: WO 2024/157711

(57) **Abstract**

A first problem of the present invention is to provide a novel technique capable of producing iPS cells using epithelial cells such as keratinocytes.

The present invention for solving the problem is a method for producing induced pluripotent stem cells using epithelial cells, the method including:
a reprogramming step including introducing a reprogramming gene for reprogramming a nucleus, or a translation product thereof, into the epithelial cells; and
an adherent culture step of adhering and culturing cells that have undergone the reprogramming step on a culture surface coated with a laminin or a fragment thereof.

## Description

### Technical Field

The present invention relates to a method for producing induced pluripotent stem cells using epithelial cells.

### Background Art

Pluripotent stem cells such as induced pluripotent stem cells (iPS cells) have been widely studied as pharmaceuticals or pharmaceutical raw materials, etc. for regenerative medicine. iPS cells were first established using mouse somatic cells in 2006 (Non Patent Literature 1), and establishment of human iPS was also reported in the following year (Non Patent Literature 2).

Currently, methods for establishing iPS cells have been developed using blood cells such as mononuclear cells derived from peripheral blood or umbilical cord blood, as well as somatic cells such as fibroblasts, and protocols have been made publicly available by Kyoto University and others.

Furthermore, a method for efficiently producing iPS cells using non-invasively acquirable somatic cells has also been studied. For example, Patent Literature 1 discloses a method for inducing iPS cells using urine-derived cells such as human exfoliated proximal tubular epithelial cells (HEPTEC) collected from urine.

Also disclosed is a method for inducing iPS cells from keratinocytes derived from an outer root sheath of a hair follicle (Non Patent Literature 3).

### Citation List

### Patent Literature

Patent Literature 1: JP 2017-169574 A

### Non Patent Literature

Non Patent Literature 1: Cell. 2006; 126(4): 663-676
Non Patent Literature 2: Cell. 2007; 131(5): 861-872
Non Patent Literature 3: Nat Protoc. 2010 Feb; 5(2): 371-82.

### Summary of Invention

### Technical Problem

In view of the above-mentioned prior art, a first problem of the present invention is to provide a novel technique capable of producing iPS cells using epithelial cells such as keratinocytes.

A second problem of the present invention is to provide a novel culture method for obtaining epithelial cells from a tissue including epithelial cells such as hair follicles.

### Solution to Problem

The present invention for solving the first problem is a method for producing induced pluripotent stem cells using epithelial cells, the method including:
a reprogramming step including introducing a reprogramming gene for reprogramming a nucleus, or a translation product thereof, into the epithelial cells; and
an adherent culture step of adhering and culturing cells that have undergone the reprogramming step on a culture surface coated with a laminin or a fragment thereof.

The production method of the present invention has an advantage that the establishment efficiency of iPS cells is high by adhering and culturing cells on the culture surface coated with a laminin or a fragment thereof in the reprogramming step.

In a preferred mode of the present invention, the epithelial cells are derived from an outer root sheath.

Since the epithelial cells derived from the outer root sheath available by a non-invasive method are used, acquisition of iPS donor cells from a donor individual is facilitated.

In a preferred mode of the present invention, the epithelial cells are derived from a tissue obtained by hair plucking.

By using the epithelial cells derived from the tissue obtained by hair plucking, iPS cells can be efficiently produced.

In a preferred mode of the present invention, the method further includes a culture step of culturing the epithelial cells used in the reprogramming step, wherein
the culture step includes a step of culturing a tissue including an outer root sheath to proliferate the epithelial cells.

By performing the culture step, the epithelial cells derived from the outer root sheath can be obtained by a non-invasive method, and it is easy to obtain iPS donor cells from a donor individual.

In a preferred mode of the present invention, the tissue including the outer root sheath is cultured in a state of being adhered to a hair.

According to the present invention, it is possible to save time and effort in sample processing such as removing the tissue including the outer root sheath from hair, and to simplify the step.

In a preferred mode of the present invention, the tissue including the outer root sheath is obtained from a hair which is obtained by plucking.

Since a culture sample can be easily obtained by hair plucking which is a non-invasive method, the burden on the donor individual at the time of obtaining the sample can be reduced.

In a preferred mode of the present invention, the reprogramming step includes a functional inhibition of p53.

By inhibiting the function of p53 during cell reprogramming, the establishment efficiency of iPS cells can be further enhanced.

In a preferred mode of the present invention, the functional inhibition of p53 is induced by one or more substances selected from (a) to (c) below:
(a) a chemical inhibitor of p53;
(b) a dominant negative mutant of p53 and a nucleic acid encoding the dominant negative mutant of p53; and
(c) a siRNA and a shRNA against p53 and a DNA encoding the siRNA and the shRNA.

In a preferred mode of the present invention, the method includes culturing cells, into which the reprogramming gene or a translation product thereof has been introduced during the reprogramming step, in a culture medium containing a ROCK inhibitor.

By adopting the above mode, the establishment efficiency of iPS cells can be further enhanced.

In a preferred mode of the present invention, the culture step includes a step of bringing the tissue including the outer root sheath, which has been obtained, into contact with an antiseptic solution for less than 1 minute before culturing.

By performing the above step, the adhesion of the hair of the sample to a cultureware can be improved, and the epithelial cells can be efficiently obtained from the outer root sheath.

In a preferred mode of the present invention, the culture step includes:
a step of seeding the tissue including the outer root sheath, which has been obtained, onto a plate coated with an extracellular matrix; and
a step of culturing the tissue including the outer root sheath seeded on the plate for 1 day to 3 days while replenishing a fresh culture medium without replacing an entire amount of a culture medium, and then subjecting the tissue including the outer root sheath to stationary culture for 2 days to 7 days without replacing or replenishing the culture medium to proliferate the epithelial cells.

By performing the culture step having the above characteristics, a large number of the epithelial cells can be efficiently obtained from a small number of hairs.

In a preferred mode of the present invention, the laminin is Laminin-511.

By using Laminin-511, the establishment efficiency of iPS cells can be enhanced.

In a preferred mode of the present invention, the reprogramming gene includes an Oct3/4 gene, a Klf4 gene, and a Sox2 gene.

In a preferred mode of the present invention, the reprogramming gene includes a c-Myc gene or an L-Myc gene.

In a preferred mode of the present invention, the epithelial cells are keratinocytes.

In a preferred mode of the present invention, a culture medium used in the culture step is serum-free and/or xeno-free.

In a preferred mode of the present invention, the culture step includes a step of culturing in a stem cell culture medium.

By including the step of culturing in the stem cell culture medium, the epithelial cells can be efficiently proliferated even under serum-free and/or xeno-free conditions.

In a preferred mode of the present invention, the culture step includes a step of culturing in an epithelial cell culture medium after culturing in the stem cell culture medium.

By adopting the above mode, the epithelial cells can be preferentially proliferated.

A preferred mode of the present invention includes: a step of culturing in a high calcium culture medium having a calcium concentration of 0.2 mM to 4.0 mM; and
a step of culturing in a low calcium culture medium having a calcium concentration of 40 µM to 80 µM after culturing in the high calcium culture medium.

By adopting the above mode, the epithelial cells can be efficiently proliferated even under serum-free and/or xeno-free conditions.

In a preferred mode of the present invention, the stem cell culture medium and/or the high calcium culture medium contains epidermal growth factor (EGF), insulin-like growth factor-I (IGF-I), hydrocortisone, transferrin, basic fibroblast growth factor (bFGF), TGFβ1 (transforming growth factor-β1), L-ascorbic acid, selenium and insulin.

By adopting the above mode, the epithelial cells can be efficiently obtained from an epithelial tissue under serum-free and/or xeno-free conditions.

In a preferred mode of the present invention, the epithelial cell culture medium and/or the low calcium culture medium contains epidermal growth factor (EGF), insulin-like growth factor-I (IGF-I), hydrocortisone, and transferrin.

By adopting the above mode, the epithelial cells can be efficiently obtained from the tissue including the outer root sheath under serum-free and/or xeno-free conditions.

In a preferred mode of the present invention, the method does not include a degradation step of degrading a component of an extracellular matrix in a tissue including the outer root sheath before seeding the tissue including the outer root sheath.

By adopting the above mode, it is possible to save time and effort in sample processing, minimize damage to the sample, and efficiently proliferate the epithelial cells from the tissue including the outer root sheath.

In a preferred mode of the present invention, the degradation step is a step of processing the tissue with one or more selected from trypsin, collagenase, and dispase.

The present invention for solving the first problem is as follows.
[1] A method for producing induced pluripotent stem cells using epithelial cells, the method including:
   a reprogramming step including introducing a reprogramming gene for reprogramming a nucleus, or a translation product thereof, into the epithelial cells; and
   an adherent culture step of adhering and culturing cells that have undergone the reprogramming step on a culture surface coated with a laminin or a fragment thereof.
[2] The method according to [1], wherein the epithelial cells are derived from an outer root sheath.
[3] The method according to [1] or [2], wherein the epithelial cells are derived from a tissue obtained by hair plucking.
[4] The method according to any one of [1] to [3], further including a culture step of culturing the epithelial cells used in the reprogramming step, wherein
   the culture step includes a step of culturing a tissue including an outer root sheath to proliferate the epithelial cells.
[5] The method according to [4], wherein the tissue including the outer root sheath is cultured in a state of being adhered to a hair.
[6] The method according to [4] or [5], wherein the tissue including the outer root sheath is obtained from a hair obtained by plucking.
[7] The method according to any one of [1] to [6], wherein the reprogramming step includes a functional inhibition of p53.
[8] The method according to [7], wherein the functional inhibition of p53 is induced by one or more substances selected from (a) to (c) below:
   (a) a chemical inhibitor of p53;
   (b) a dominant negative mutant of p53 and a nucleic acid encoding the dominant negative mutant of p53; and
   (c) a siRNA and a shRNA against p53 and a DNA encoding the siRNA and the shRNA.
[9] The method according to any one of [1] to [8], wherein the method includes culturing cells, into which the reprogramming gene or a translation product thereof has been introduced during the reprogramming step, in a culture medium containing a ROCK inhibitor.
[10] The method according to any one of [4] to [9], wherein the culture step includes a step of bringing the tissue including the outer root sheath, which has been obtained, into contact with an antiseptic solution for less than 1 minute before culturing.
[11] The method according to any one of [4] to [10], wherein the culture step includes: a step of seeding the tissue including the outer root sheath, which has been obtained, onto a plate coated with an extracellular matrix; and
   a step of culturing the tissue including the outer root sheath seeded on the plate for 1 day to 3 days while replenishing a fresh culture medium without replacing an entire amount of a culture medium, and then subjecting the tissue including the outer root sheath to stationary culture for 2 days to 7 days without replacing or replenishing the culture medium to proliferate the epithelial cells.
[12] The method according to any one of [1] to [11], wherein the laminin is Laminin-511.
[13] The method according to any one of [1] to [12], wherein the reprogramming gene includes an Oct3/4 gene, a Klf4 gene, and a Sox2 gene.
[14] The method according to any one of [1] to [13], wherein the reprogramming gene includes a c-Myc gene or an L-Myc gene.
[15] The method according to any one of [1] to [14], wherein the epithelial cells are keratinocytes.
[16] The method according to any one of [4] to [15], wherein a culture medium used in the culture step is serum-free and/or xeno-free.
[17] The method according to [16], wherein the culture step includes a step of culturing in a stem cell culture medium.
[18] The method according to [17], wherein the culture step includes a step of culturing in an epithelial cell culture medium after culturing in the stem cell culture medium.
[19] The method according to any one of [16] to [18], wherein the culture step includes: a step of culturing in a high calcium culture medium having a calcium concentration of 0.2 mM to 4.0 mM; and
   a step of culturing in a low calcium culture medium having a calcium concentration of 40 µM to 80 µM after culturing in the high calcium culture medium.
[20] The method according to any one of [17] to [19], wherein the stem cell culture medium and/or the high calcium culture medium contains epidermal growth factor (EGF), insulin-like growth factor-I (IGF-I), hydrocortisone, transferrin, basic fibroblast growth factor (bFGF), TGFβ1 (transforming growth factor-β1), L-ascorbic acid, selenium and insulin.
[21] The method according to any one of [18] to [20], wherein the epithelial cell culture medium and/or the low calcium culture medium contains epidermal growth factor (EGF), insulin-like growth factor-I (IGF-I), hydrocortisone, and transferrin.
[22] The method according to any one of [4] to [21], wherein the method does not include a degradation step of degrading a component of an extracellular matrix in a tissue including the outer root sheath before seeding the tissue including the outer root sheath.
[23] The method according to [22], wherein the degradation step is a step of processing the sample with one or more selected from trypsin, collagenase, and dispase.

The present invention for solving the second problem is a method for proliferating epithelial cells from a tissue under serum-free and/or xeno-free conditions.

Preferred modes of the present invention are as follows.

[1] A method for culturing epithelial cells, the method including: culturing a sample containing an epithelial tissue under serum-free and/or xeno-free conditions.
[2] The method according to [1], wherein the epithelial cells are keratinocytes.
[3] The method according to [2], wherein the keratinocytes are hair follicle keratinocytes.
[4] The method according to any one of [1] to [3], wherein the sample includes an outer root sheath.
[5] The method according to [4], wherein the outer root sheath is in a state of being adhered to the hair.
[6] The method according to any one of [1] to [5], wherein the sample is obtained from a hair obtained by plucking.
[7] The method according to any one of [1] to [6], further including a step of culturing the sample in a stem cell culture medium.
[8] The method according to [7], wherein the stem cell culture medium has a calcium concentration of 0.2 mM to 4.0 mM.
[9] The method according to [7] or [8], further including a step of culturing the sample in an epithelial cell culture medium after culturing the sample in the stem cell culture medium.
[10] The method according to any one of [1] to [9], further including: a step of culturing the sample in a high calcium culture medium having a calcium concentration of 0.2 mM to 4.0 mM; and
   a step of culturing the sample in a low calcium culture medium having a calcium concentration of 40 µM to 80 µM after culturing the sample in the high calcium culture medium.
[11] The method according to [10], wherein the high calcium culture medium is a stem cell culture medium.
[12] The method according to [10] or [11], wherein the low calcium culture medium is an epithelial cell culture medium.
[13] The method according to any one of [7] to [12], wherein the stem cell culture medium and/or the high calcium culture medium contains epidermal growth factor (EGF), insulin-like growth factor-I (IGF-I), hydrocortisone, transferrin, basic fibroblast growth factor (bFGF), TGFβ1, L-ascorbic acid, selenium, and insulin.
[14] The method according to any one of [9] to [13], wherein the epithelial cell culture medium and/or the low calcium culture medium contains epidermal growth factor (EGF), insulin-like growth factor-I (IGF-I), hydrocortisone, and transferrin.
[15] The method according to any one of [1] to [14], wherein the sample is cultured under serum-free and xeno-free conditions.
[16] The method according to any one of [1] to [15], wherein the method for culturing the epithelial cells is a method of primary culture.
[17] The method according to any one of [1] to [16], wherein the method does not include a degradation step of degrading a component of an extracellular matrix in the sample before seeding the sample.
[18] The method according to [17], wherein the degradation step is a step of processing the sample with one or more selected from trypsin, collagenase, and dispase.
[19] The method according to any one of [1] to [18], further including a step of bringing the sample into contact with an antiseptic solution for less than 1 minute before culturing.
[20] The method according to any one of [1] to [19], further including: a step of seeding the sample, which has been obtained, onto a plate coated with an extracellular matrix; and
   a stationary culture step of culturing the sample seeded on the plate for 1 day to 3 days while replenishing a fresh culture medium without replacing an entire amount of a culture medium, and then subjecting the sample to stationary culture for 2 days to 7 days without replacing or replenishing the culture medium to proliferate the epithelial cells.
[21] The method according to [20], wherein the culture medium used in the stationary culture step is the stem cell culture medium.
[22] The method according to [21], wherein the stem cell culture medium has a calcium concentration of 0.2 mM to 4.0 mM.

### Advantageous Effects of Invention

According to the production method of the present invention, iPS cells derived from epithelial cells can be efficiently produced.

According to the production method of the present invention, epithelial cells can be obtained from a tissue including epithelial cells such as hair follicles under serum-free and/or xeno-free conditions.

### Brief Description of Drawings

Fig. 1 is a process chart for describing steps for producing iPS cells using epithelial cells according to one embodiment of the present invention.
Fig. 2 is a bright field photograph showing a state of epithelial cells proliferated from an outer root sheath according to Example 1. Here, scale bar is 1 mm.
Fig. 3 is a bright field photograph showing a state of proliferation of iPS cells 20 days after subculture following transformation according to Example 1. Here, scale bar is 200 µm.
Fig. 4 is a fluorescent photograph showing immunostaining results for iPS cell markers (OCT4, SOX2, NANOG, and TRA1-81) in the iPS cells induced in Example 1. Here, scale bar is 200 µm.
Fig. 5 is a bright field photograph showing a state of epithelial cells proliferated from an outer root sheath according to Example 2. Here, scale bar is 300 µm.
Fig. 6 is a bright field photograph showing a state of proliferation of iPS cells derived from epithelial cells according to Example 2. Here, scale bar is 100 µm.
Fig. 7 is a fluorescent photograph showing immunostaining results for iPS cell markers (OCT4, SOX2, NANOG, and TRA1-81) in the iPS cells induced in Example 2. Here, scale bar is 100 µm.
Fig. 8 is a fluorescent photograph showing a result of cellular immunostaining of epithelial cell markers (KRT14 and CD200) of epithelial cells obtained in Example 1 according to Example 3. Here, scale bar is 500 µm.

### Description of Embodiments

### <Method for producing induced pluripotent stem cells using epithelial cells>

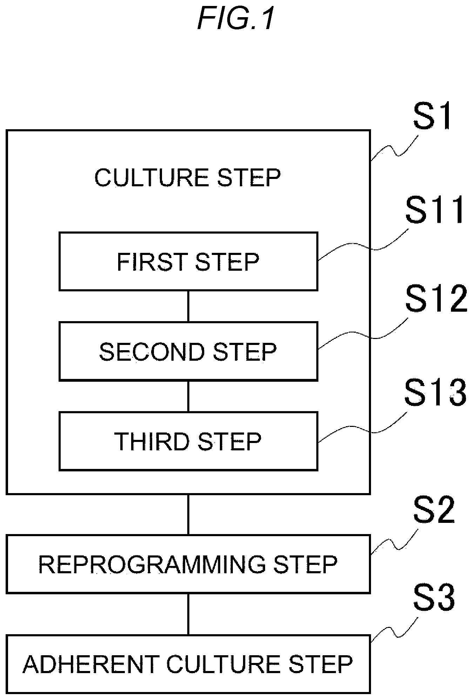

One preferred embodiment of the method for producing induced pluripotent stem cells using epithelial cells according to the present invention will be described with reference to Fig. 1.

Note that the present invention is not limited to the present embodiment, and can be modified in design as appropriate.

The production method according to Fig. 1 includes a culture step S1 of culturing epithelial cells, a reprogramming step S2 of reprogramming the obtained epithelial cells, and an adherent culture step S3 of inducing induced pluripotent stem cells (iPS cells) by adhering and culturing the epithelial cells on a culture surface coated with a laminin or a fragment thereof.

Here in the present invention, the induced pluripotent stem cells (iPS cells) refer to cells having pluripotency and proliferation capability, which are artificially produced by causing a reprogramming factor described later to act on somatic cells.

Hereinafter, each step according to the present invention will be described in detail.

### (1) Culture step S1

The culture step S1 is a step of culturing the epithelial cells to be used in the reprogramming step S2.

As the epithelial cells used in the present invention, those derived from mammals (for example, human and the like) can be used. In the present invention, it is preferable to culture human-derived epithelial cells.

In the present invention, the epithelial cells refer to cells derived from an epithelial tissue, and can be obtained by culturing a sample containing an epithelial tissue. The epithelial cells in the present invention may be a cell population obtained by culturing an epithelial tissue, and may include epithelial cells expressing an epithelial cell marker (for example, keratinocytes) and stem cells expressing an epithelial stem cell marker (for example, hair follicle stem cells (HFSC)).

In the present invention, keratinocytes can be used as epithelial cells, and among them, hair follicle keratinocytes can be suitably used.

The epithelial cells used in the present invention can be obtained by culturing specimens having epithelial cells, that is, specimens such as skin and skin appendages. In the present invention, it is preferable to culture cells of an outer root sheath of hair for invasiveness reasons.

In a preferred embodiment of the present invention, epithelial cells derived from the outer root sheath of hair available by a non-invasive method are used, and thus, it is possible to reduce the burden when collecting a sample for iPS cell induction from a donor individual.

The individual (specifically, a mammalian individual) from which the tissue for obtaining epithelial cells is collected is not particularly limited. For example, when iPS cells are used as a source of cells for screening for evaluating the cosmetic effect, drug sensitivity, the presence or absence of side effects, and the like of a donor individual, it is preferable to collect epithelial cells from the donor himself/herself or another person having the same genetic polymorphism correlated with the drug sensitivity and the like as the donor.

In addition, when epithelial cells are to be used for transplantation into a patient or the like, it is preferable to collect the epithelial cells from the donor himself/herself such as the patient or another person whose HLA type is the same or substantially the same as that of the donor. As a result, the risk of rejection is avoided, and thus the produced iPS cells can be suitably used for regenerative medicine.

Here, the expression that the types of HLA are "substantially the same" means that the types of HLA are matched to such an extent that transplanted cells can engraft when the cells obtained by inducing differentiation from iPS cells derived from somatic cells by using an immunosuppressive agent or the like are transplanted to a donor individual. Examples thereof include a case where HLAs (three loci, e.g., HLA-A, HLA-B, and HLA-DR) are identical.

The culture medium used for culturing epithelial cells is not particularly limited, and the epithelial cells can be cultured using a known culture medium suitable for culturing the epithelial cells such as keratinocytes. Examples of such culture medium include, but are not limited to, EpiLife^{™} Medium (Gibco), Keratinocyte Growth Medium, Minimal Essential Medium (MEM) containing about 5% to 20% fetal bovine serum, Dulbecco's Modified Eagle's Medium (DMEM), RPMI1640 culture medium, 199 culture medium, F12 culture medium. In addition, a component suitable for culturing keratinocytes (calcium, growth factors, etc.) may be appropriately added.

In the present invention, it is preferable to use a culture medium containing calcium salt of 40 µM to 400 µM.

In order to prevent a decrease in the introduction efficiency, it is preferable to replace the culture medium with a serum-free culture medium before the reprogramming step S2.

It is preferable that the culture of epithelial cells is adherent culture using a culture vessel to which cells can adhere.

An appropriate culture vessel can be appropriately selected as the culture vessel used for the adherent culture according to the culture scale, the culture conditions, and the culture period. Examples of the culture vessel having cell adhesiveness include a culture vessel in which the surface of the culture vessel is artificially processed for the purpose of improving adhesion with cells, and specifically include a culture vessel in which the surface is processed and a culture vessel in which the inside is coated with a coating agent.

In the present invention, it is preferable to use a culture vessel coated with a coating agent for culturing epithelial cells.

Examples of the coating agent include extracellular matrixes such as laminin, entactin, collagen, gelatin, vitronectin, syndemax (Corning Incorporated), and matrigel, polymers such as polylysine and polyornithine, etc. In the present invention, it is preferable to use an extracellular matrix, and it is preferable to use collagen I.

In the present invention, it is also preferable to use a laminin or a fragment thereof as the coating agent. The preferred form of the laminin or the fragment thereof is as described in the adherent culture step S3 described later.

Examples of specific forms of the culture vessel include a flask, a tissue culture flask, a culture dish (dish), a tissue culture dish, a multi-dish, a microplate, a microwell plate, a multi-plate, a multi-well plate, a chamber slide, a petri dish, a tube, a tray, a culture bag, a microcarrier, a bead, a stack plate, a spinner flask, and a roller bottle.

Subsequently, as a specific procedure of the culture step S1, a method for separating and culturing epithelial cells using hair will be described.

First, a tissue including an outer root sheath is obtained. The outer root sheath is a layer constituting an epithelial root sheath of a hair follicle, and the tissue including the outer root sheath can be obtained by plucking hairs of a donor individual. The number of hairs used in the present invention may be at least one, and is preferably two or more, more preferably five or more. Specifically, it is preferable to use 1 to 10 hairs. Then, the lower portion of each hair collected from the donor individual is cut out to obtain the tissue including the outer root sheath. It is preferable that the size of the tissue obtained is 2 mm to 3 mm or more. The tissue obtained may be in a form in which the outer root sheath remains adhered to the hair. That is, the tissue obtained may be in a form in which the portion including the outer root sheath is cut out from the plucked hair to obtain a hair follicle including the outer root sheath adhered to the hair.

In the hair obtained by plucking, the outer root sheath is adhered to a region above a hair bulb. The tissue obtained by plucking to be cultured in the present invention may contain cells present in a bulge region.

The obtained tissue (sample) including the outer root sheath is then brought into contact with an antiseptic solution. The method for bringing the sample into contact with the antiseptic solution is not particularly limited, and it is preferable to immerse the sample in the antiseptic solution.

The time for contact with the antiseptic solution is preferably less than 1 minute, more preferably less than 30 seconds, still more preferably less than 10 seconds, and particularly preferably less than 5 seconds.

By setting the time of contact with the antiseptic solution within the above range, damage to the hair is reduced and adhesion of the hair to the cultureware is improved, whereby epithelial cells can be efficiently obtained from the tissue including the outer root sheath.

It is preferable that the antiseptic solution used in the present invention is a solution to which an antibiotic is added. Preferred antibiotics are one or more selected from cell wall synthesis inhibitors, protein synthesis inhibitors, and cell membrane function inhibitors. More preferably, one or more antibiotics are selected from each of cell wall synthesis inhibitors, protein synthesis inhibitors, and cell membrane function inhibitors.

Preferred examples of cell wall synthesis inhibitors include β-lactam antibiotics. Examples of β-lactam cell wall synthesis inhibitors include penicillin, ampicillin, and carbenicillin sodium, and in the present invention, it is preferable to use penicillin.

Preferred examples of protein synthesis inhibitors include aminoglycoside antibiotics.

Examples of aminoglycoside protein synthesis inhibitors include kanamycin, gentamicin, streptomycin, neomycin, hygromycin, and tobramycin, and in the present invention, it is preferable to use streptomycin.

Preferable examples of cell membrane function inhibitors include polyene antibiotics. Examples of polyene cell membrane function inhibitors include amphotericin B, nystatin, and natamycin, and in the present invention, it is particularly preferable to use amphotericin B.

It is preferable that the antiseptic solution according to the present invention is a solution containing penicillin, streptomycin, and amphotericin B as antibiotics.

Subsequently, the obtained disinfected sample is seeded on a plate coated with a coating agent, and cultured for a certain period of time to proliferate epithelial cells.

The plate to be used is preferably a 12-well plate or a 24-well plate for cell culture, and more preferably a 24-well plate. In addition, it is preferable to seed disinfected samples one by one per well.

It is preferable that the amount of the culture medium at the time of seeding is an amount at which the entire tissue of the cut sample is immersed and the liquid level is 1 mm or less above the entire tissue. By setting the culture medium at the time of seeding to the above amount, a hair follicle tissue can be efficiently adhered to the plate.

In a preferred embodiment, the culture of the sample including the outer root sheath includes the following three steps in sequence.

In the first step S11, the seeded sample is cultured while replenishing a fresh culture medium without replacing the entire amount of the culture medium. The number of times of replenishment of the culture medium is preferably 1 to 3 times, and more preferably 2 times.

It is preferable that the amount of the culture medium replenished in the first step S11 is an amount at which the entire tissue of the cut sample is immersed and the liquid level is 2 mm or less, more preferably 1.5 mm or less above the entire tissue.

In addition, the amount of the culture medium to be replenished is preferably 0.5 to 3 times, more preferably 1 to 3 times, and still more preferably 1 to 2 times the amount at the time of seeding the sample. When the number of times of replenishment of the culture medium is two or more, it is preferable to increase the replenishment amount of the culture medium for the second and subsequent times as compared with the replenishment amount of the culture medium for the first time. In such a case, the replenishment amount of the culture medium for the second and subsequent times is preferably 1.2 to 3 times, more preferably 1.5 to 2.5 times, and still more preferably 2 to 2.5 times the replenishment amount of the culture medium of the first time. In addition, it is preferable that the replenishment amount of the culture medium for the second and subsequent times is an amount at which the entire cut tissue is immersed and the liquid level is 1 to 2 mm, more preferably 1 to 1.5 mm above the entire tissue.

The culture period in the first step S11 is preferably 1 day to 3 days.

As a specific mode of the first step S11, a mode, in which the number of times the culture medium is replenished is 2, the culture period is 3 days, the culture medium is replenished once during 24 hours to 48 hours after seeding the sample by an equal amount (1 time) of the culture medium at the time of seeding, and the culture medium is replenished once during 48 hours to 72 hours after seeding the sample by two times the amount of the culture medium at the time of seeding, can be preferably exemplified.

In the second step S12, after the culture medium is replenished in the first step S11, stationary culture is performed without replacement or replenishment of the culture medium to proliferate epithelial cells. The culture period is preferably 2 days to 7 days, more preferably 3 days to 5 days, and still more preferably 3 days to 4 days. Specifically, it is preferable that the period is 3 days.

By performing such stationary culture, proliferation of epithelial cells from the outer root sheath can be promoted.

In addition, in the second step S12, expansion of the cell population of epithelial cells from the seeded outer root sheath can be confirmed. The expansion distance of the epithelial cells proliferated from the outer root sheath is preferably 0.8 mm or more from a hair shaft, more preferably 1 mm or more from the hair shaft, and specifically, it is preferable that the expansion distance is 1 mm to 2 mm from the hair shaft.

The step of sequentially performing the first step S11 and the second step S12 is also referred to as stationary culture step in the present invention.

In the third step S13, the culture medium is replaced, and the culture of the cells expanded from the outer root sheath is continued. It is preferable that the entire amount of the culture medium is replaced at the first time after the second step S12, and thereafter, the replenishment and replacement of the culture medium are repeated. It is preferable that the replenishment and replacement of the culture medium are performed alternately at intervals of preferably 45 hours to 55 hours, more preferably 48 hours to 50 hours, and specifically, it is preferable that the replenishment and replacement of the culture medium are performed alternately every other day.

In the third step S13, the addition amount of the culture medium at the time of culture medium replacement is preferably 0.5 to 3 times, more preferably 1 to 2 times, and still more preferably 1.5 to 2 times the amount at the time of seeding the sample before the first step S11.

In addition, the replenishment amount of the culture medium is preferably 0.5 to 2 times, more preferably 1 to 1.5 times, and still more preferably equal to (1 time) the addition amount of the culture medium at the time of culture medium replacement in the third step S13.

As a specific mode of the third step S13, a mode can be preferably exemplified in which the total amount of the culture medium is replaced, 2 times the amount of the culture medium at the time of seeding the sample is added, and 2 days later, the same amount (1 time) of the culture medium as the addition amount of the culture medium at the time of culture medium replacement is added to perform culture.

It is preferable that the third step S13 is continued until the proliferated cells are 50% to 80% confluent. The culture period with replenishment and replacement of the culture medium is preferably 7 days to 28 days, and more preferably 14 days to 21 days.

In addition, in the third step S13, it is preferable to perform subculture before the cells become overcrowded in a developed region of the cells proliferated from the outer root sheath. For example, when culture is performed in a 12-well plate (well bottom diameter being 21 mm to 22.3 mm), it is preferable that subculture is performed at a stage when the proliferated cells are 50% to 80% confluent. The cells which are 50% to 80% confluent after the subculture can be suitably used in the subsequent reprogramming step S2.

The culture period after the subculture is preferably 3 days to 7 days, and more preferably 4 days to 6 days. In addition, it is preferable to replace the culture medium every day after the subculture, and the amount of the culture medium at the time of replacement is preferably 0.5 to 2 times, more preferably 1 to 1.5 times, and still more preferably equal to (1 time) the addition amount of the culture medium at the time of culture medium replacement in the third step S13.

By adopting the above three steps, it is possible to obtain a sufficient amount of epithelial cells for inducing iPS cells from a small number of hairs. Therefore, by using the present invention, it is possible to reduce the burden when collecting a sample from a donor individual.

In one embodiment, in the first step S11 and the second step S12, it is preferable to use a mixture of a calcium-containing culture medium and a MEF-conditioned medium, and specific examples thereof include a mixed culture medium of EpiLife^{™} Medium (with a calcium concentration of 60 µM, Gibco) and EmbryoMax MEF-conditioned media (Merck). In addition, it is preferable that the concentration of the calcium salt in the culture medium used in the first step S11 and the second step S12 is 60 µM to 400 µM.

In the third step S13, it is preferable to use a calcium-containing culture medium, and specific examples thereof include EpiLife^{™} Medium (with a calcium concentration of 60 µM, Gibco). It is preferable that the concentration of the calcium salt in the culture medium used in the third step S13 is 40 µM to 80 µM.

It is preferable that the culture medium used in the first step S11 to the third step S13 contains bovine pituitary extract (BPE), recombinant human insulin-like growth factor-I, hydrocortisone, bovine transferrin, and human epidermal growth factor.

In addition, when the size of the cells expanded from the outer root sheath is less than or equal to a specified value, for example, 1 mm or less, it is preferable to include a step of culturing using the MEF-conditioned medium before proceeding to the third step S13. In this case, it is preferable that the MEF-conditioned medium contains bovine pituitary extract (BPE), recombinant human insulin-like growth factor-I, hydrocortisone, bovine transferrin and human epidermal growth factor.

In another embodiment, it is preferable that the culture medium used in the culture step S1 is serum-free or xeno-free. It is more preferable that the culture medium used in the culture step S1 is serum-free and xeno-free.

The serum-free and/or xeno-free culture medium preferably contains one or more additives selected from epidermal growth factor (EGF), insulin-like growth factor-I (IGF-I), hydrocortisone, and transferrin, and more preferably contains all of the above four additives.

By the culture medium containing these components, epithelial cells can be efficiently proliferated.

The concentration of EGF is preferably 5 ng/ml to 50 ng/ml, more preferably 10 ng/ml to 30 ng/ml, further preferably 15 ng/ml to 25 ng/ml, and specifically preferably 20 ng/ml.

It is preferable that EGF is a human epidermal growth factor.

The concentration of IGF-I is preferably 5 ng/ml to 50 ng/ml, more preferably 10 ng/ml to 30 ng/ml, further preferably 15 ng/ml to 25 ng/ml, and specifically preferably 20 ng/ml.

In addition, it is preferable that IGF-I is a recombinant human insulin-like growth factor.

The concentration of hydrocortisone is preferably 50 ng/ml to 500 ng/ml, more preferably 100 ng/ml to 300 ng/ml, further preferably 150 ng/ml to 250 ng/ml, and specifically preferably 200 ng/ml.

The concentration of transferrin is preferably 1 µg/ml to 50 µg/ml, more preferably 3 ug/ml to 30 µg/ml, further preferably 5 ug/ml to 20 µg/ml, and specifically preferably 10 ug/ml.

It is preferable that the transferrin is a human transferrin.

In addition, the serum-free and/or xeno-free culture medium may contain any proliferation additive for epithelial cells. The epithelial cell additive is preferably a xeno-free component, and Supplement S7 (Gibco) can be preferably exemplified.

When a serum-free and/or xeno-free culture medium is used, it is preferable that the culture step S1 includes a step of culturing in a stem cell culture medium.

Suitable examples of the stem cell culture medium to be used include Essential 8, StemPro (manufactured by Life Technologies Corporation), mTeSR1 (manufactured by STEMCELL Technologies), mTeSR2 (manufactured by STEMCELL Technologies), TeSR-E8 (manufactured by STEMCELL Technologies), and StemFit (manufactured by Ajinomoto Co., Inc.), and among them, it is preferable to use Essential 8.

Additives such as antibiotics, serum, growth factors, and hormones may be optionally added to the stem cell culture medium.

More preferably, it is preferable to include a step of culturing in the stem cell culture medium and then culturing in an epithelial cell culture medium.

As the epithelial cell culture medium to be used, a culture medium suitable for culturing epithelial cells described in the culture step S1 can be suitably used, and it is preferable to use EpiLife^{™} Medium (Gibco) and HuMedia-KG2 (KURABO), and more preferable to use EpiLife^{™} Medium. Additives such as antibiotics, serum, growth factors, hormones, and calcium may be optionally added to the epithelial cell culture medium.

When a serum-free and/or xeno-free culture medium is used, it is preferable that the culture step S1 includes a step of culturing in a culture medium having a high calcium concentration (high calcium culture medium).

When a serum-free and/or xeno-free culture medium is used, it is preferable to use two types of culture medium having different calcium concentrations. In one embodiment, it is preferred to culture in a high calcium culture medium and then in a culture medium having a low calcium concentration (low calcium culture medium).

The high calcium culture medium has a calcium concentration of preferably 0.2 mM or more, more preferably 0.5 mM or more, still more preferably 0.8 mM or more, and further more preferably 1.0 mM or more.

In addition, the high calcium culture medium has a calcium concentration of preferably 4.0 mM or less, more preferably 3.5 mM or less, and still more preferably 3.0 mM or less.

By setting the calcium concentration of the high calcium culture medium within the above range, epithelial cells can be efficiently proliferated from the outer root sheath after seeding.

The concentration of the high calcium culture medium can be specifically set to 0.2 mM to 4.0 mM, more preferably 0.5 mM to 3.5 mM, still more preferably 1.0 mM to 3.5 mM, and further more preferably 1.0 mM to 3.0 mM.

The low calcium culture medium has a calcium concentration of preferably 40 µM or more, more preferably 45 µM or more, and still more preferably 50 µM or more.

In addition, the low calcium culture medium has a calcium concentration of preferably 80 µM or less, more preferably 75 µM or less, and still more preferably 70 µM or less.

By setting the calcium concentration of the low calcium culture medium within the above range, the target epithelial cells can be efficiently proliferated from the cells proliferated by culturing in the high calcium culture medium.

The concentration of the low calcium culture medium can be specifically set to 40 µM to 80 µM, more preferably 45 µM to 75 µM, and still more preferably 50 µM to 70 µM.

Subsequently, preferred embodiments of the serum-free and xeno-free culture medium will be described with specific examples. In the present embodiment, the culture step S1 includes a primary cultivation step of culturing using a primary culture medium which is a high calcium culture medium and is a stem cell culture medium, and a secondary cultivation step of culturing using a secondary culture medium which is a low calcium culture medium and is an epithelial cell culture medium.

It is preferable that a basal culture medium is DMEM/F12 in the primary culture medium. It is preferable that the primary culture medium contains basic fibroblast growth factor (bFGF) and/or transforming growth factor-β1 (TGFβ1). By the primary culture medium containing such a component, cells can be efficiently proliferated from the outer root sheath of hair.

Furthermore, it is preferable that the primary culture medium contains L-ascorbic acid, selenium, transferrin and insulin. Examples of the primary culture medium containing such a component include Essential 8.

It is preferable that the primary culture medium contains, in the above-described culture medium, one or more additives selected from epidermal growth factor (EGF), insulin-like growth factor-I (IGF-I), hydrocortisone and transferrin, and it is more preferable that the primary culture medium contains all of the four additives. Preferred forms of the additive are as described above.

In the present invention, the primary culture medium can be obtained by adding the above four additives to Essential 8. Preferred calcium concentrations are as described under conditions of high calcium culture medium.

In the secondary culture medium, it is preferable to use EpiLife^{™} Medium (with a calcium concentration of 60 µM, Gibco) as the epithelial cell culture medium.

It is preferable that the secondary culture medium contains, in the above-described culture medium, one or more additives selected from epidermal growth factor (EGF), insulin-like growth factor-I (IGF-I), hydrocortisone and transferrin, and it is more preferable that the secondary culture medium contains all of the four additives. Preferred forms of the additive are as described above.

In the primary cultivation step, it is preferable to perform the first step S11 and the second step S12 using the primary culture medium. Preferred modes of the first step S11 and the second step S12 are as described above.

In the primary cultivation step, it is preferable to replace the culture medium with the primary culture medium after performing the second step S12 and continue the culture. In the culture medium replacement of the primary culture medium, the replacement amount of the culture medium is preferably 0.5 to 2 times, more preferably 1 to 1.5 times, and still more preferably equal to (1 time) the addition amount at the time of seeding.

The culture period of the primary cultivation step is preferably 10 days or longer, more preferably 14 days or longer, and still more preferably 18 days or longer. In addition, the culture period of the primary cultivation step is preferably 30 days or less, more preferably 25 days or less, and still more preferably 22 days or less.

By setting the culture period of the primary cultivation step within the above range, epithelial cells can be efficiently obtained from the tissue including the outer root sheath.

For example, the culture period of the primary cultivation step can be 10 days to 30 days, more preferably 14 days to 25 days, and still more preferably 18 days to 22 days.

In the secondary cultivation step, after the primary cultivation step, culture is performed using the secondary culture medium.

Preferably, in the primary cultivation step, the cells expanded from the outer root sheath grow to a specified size, for example, 1 mm or more, preferably 1 mm to 2 mm, and then the secondary cultivation step is performed.

In the secondary cultivation step, it is preferable to perform the third step S13 using the secondary culture medium.

The culture period of the secondary cultivation step is preferably 5 days or longer, more preferably 7 days or longer, and still more preferably 10 days or longer. In addition, the culture period of the secondary cultivation step is preferably 20 days or less, more preferably 18 days or less, and still more preferably 16 days or less.

By setting the culture period of the secondary cultivation step within the above range, keratinocytes from the outer root sheath can be efficiently obtained.

For example, the culture period of the secondary cultivation step can be 5 days to 20 days, more preferably 7 days to 18 days, and still more preferably 10 days to 16 days.

In the secondary cultivation step, it is preferable to replace and replenish the culture medium in accordance with the proliferation of the cells.

In addition, the secondary cultivation step may include a step of performing subculture.

Preferred embodiments of replacement, replenishment, and subculture of the culture medium are as described above in the third step S13. In addition, it is also preferable that the third step S13 in the secondary cultivation step is not replenishment but total amount replacement of a culture solution.

In addition, it is preferable that the present invention does not include a degradation step of degrading the components of the extracellular matrix for the sample before seeding the tissue including the outer root sheath.

In the present invention, a mode not including the step of processing the sample with one or more selected from trypsin, collagenase, and dispase before seeding the sample containing the epithelial tissue is preferable, and a mode not including the step of processing with trypsin, collagenase, and dispase is more preferable.

By not degrading such an extracellular matrix, it is possible to save time and effort in sample processing, minimize damage to the sample, and efficiently proliferate the epithelial cells according to the present invention from the tissue including the outer root sheath.

In the above embodiment, the mode including the culture step S1 of culturing epithelial cells has been exemplified, but the present invention may be performed from the reprogramming step S2 of (2) described later. In this case, commercially available epithelial cells such as keratinocytes can be used for inducing iPS cells. Examples of such keratinocytes include normal human epidermal keratinocytes (NHEK) (manufactured by PromoCell GmbH) and hair follicular keratinocytes (HHFK) (manufactured by ScienCell Research Laboratories, Inc.).

### (2) Reprogramming step S2

The reprogramming step S2 includes a step of introducing a reprogramming gene for reprogramming a nucleus or a translation product thereof into epithelial cells, and a step of culturing cells after introduction of the reprogramming gene or the like.

The reprogramming gene in the present invention refers to a gene encoding a proteinaceous factor (reprogramming factor) capable of inducing iPS cells from somatic cells. Such reprogramming genes are, for example, one or more genes selected from a group consisting of members of the Oct family, the Klf family, the Sox family, the Myc family, the Lin family, and the Glis family.

It is preferable that the reprogramming gene according to the present invention contains at least Oct3/4, Klf4 and Sox2. Furthermore, it is preferable that the reprogramming gene according to the present invention contains one or more selected from c-Myc, L-Myc, Lin28, and Lin28b, and it is more preferable to further combine c-Myc or L-Myc with Lin28 or Lin28b.

In a preferred mode of the present invention, the combination of reprogramming genes is Oct3/4, Klf4, Sox2, L-Myc and Lin28.

In the present invention, a nucleic acid encoding a reprogramming gene may be introduced into epithelial cells, or a translation product (protein) of a reprogramming gene may be introduced into epithelial cells. The nucleic acid encoding the reprogramming gene may be in any form of DNA or RNA.

A method for introducing a reprogramming gene or a translation product thereof into epithelial cells is not particularly limited. Examples of the introduction method include infection with a viral vector such as a retroviral vector, a lentiviral vector, a sendaiviral vector, an adenoviral vector, or an adeno-associated viral vector, gene introduction by a calcium phosphate method, a lipofection method, an electroporation method, or the like of various vectors (plasmid vectors, episomal vectors, RNA vectors, etc.), and direct introduction by microinjection.

In the present invention, it is preferable to introduce a DNA encoding a reprogramming gene using an episomal vector. It is preferable that the introduction of the episomal vector is performed by a lipofection method.

In the reprogramming step S2, it is preferable to inhibit the function of p53 together with the introduction of the reprogramming gene or the like.

In the present invention, "inhibiting the function of p53" refers to inhibiting the function of p53 protein by directly acting on p53 protein, or inhibiting the function of p53 protein or expression of p53 gene by acting on a factor involved in signal transduction of p53 protein.

Methods for inhibiting the function of p53 include, but are not limited to, contacting cells with a chemical inhibitor of p53, a dominant negative mutant of p53, an anti-p53 antagonist antibody, a decoy nucleic acid containing a consensus sequence of a p53 response element, a substance that inhibits the p53 pathway, or the like.

In the present invention, inhibition of the function of p53 can be induced by introducing one or more substances selected from (a) a chemical inhibitor of p53, (b) a dominant negative mutant of p53 and a nucleic acid encoding the dominant negative mutant of p53, and (c) siRNA and shRNA for p53, and DNA encoding the siRNA and shRNA for p53.

For specific examples of the function inhibitor of p53, methods for obtaining them, and methods for contacting with somatic cells, WO 2009/157593, etc. can be referred to.

(a) Examples of the chemical inhibitor of p53 include small molecules such as Pifithrin-α (PFT-α).
(b) Preferred examples of the dominant negative mutant of p53 include p53DD in which the amino acid at position 14 to 301 (corresponding to position 11 to 304 in human p53) of mouse p53 is deleted (Bowman, T., Genes Develop., 10, 826-835 (1996)).
(c) The siRNA and shRNA for p53 can be designed by existing methods based on mouse or human p53 cDNA sequence information.

In the present invention, it is preferable to inhibit the function of p53 by introducing one or more substances selected from (b) the dominant negative mutant of p53 and the nucleic acid encoding the dominant negative mutant of p53 and (c) the siRNA and shRNA for p53, and the DNA encoding the siRNA and shRNA for p53.

In a more preferred embodiment, a vector incorporating (b) the DNA encoding the dominant negative mutant of p53 or (c) the DNA encoding the siRNA and shRNA for p53 is introduced into cells to inhibit the function of p53.

In the present invention, a p53-related gene sequence and the sequence of the reprogramming gene may be encoded in one vector, or the p53-related gene sequence and the sequence of the reprogramming gene may be encoded in different vectors. In the latter case, it is preferable that the vector encoding the p53-related gene sequence and the vector encoding the sequence of the reprogramming gene are mixed and simultaneously introduced into the cells.

In the present invention, it is preferable to use an episomal vector having a DNA sequence encoding a dominant negative mutant of p53 (p53DD).

Here, in a preferred embodiment, the episomal vector to be used contains an EBNA-1 gene sequence. Furthermore, it is preferable that the reprogramming factor and p53DD are controlled by a CAG promoter.

In addition, it is preferable that the episomal vector used in the present invention is a vector carrying an expression cassette containing one type of reprogramming gene or a combination of a plurality of reprogramming genes. Preferable examples of the expression cassette include (i) an expression cassette containing a nucleic acid encoding Oct3/4, (ii) an expression cassette containing a nucleic acid encoding Sox2 and a nucleic acid encoding Klf4, (iii) an expression cassette containing a nucleic acid encoding L-Myc and a nucleic acid encoding Lin28, and (iV) an expression cassette containing a nucleic acid encoding mmp53DD. Preferable examples of the vector carrying the expression cassette include pCE-hOCT3/4 or pCE-hOCT3/4-shp53-F encoding human OCT3/4, pCE-hSK encoding human Sox2 and human Klf4, pCE-hUL encoding human L-Myc and human LIN28, and pCE-mp53DD encoding mouse p53DD. In the present invention, it is preferable to introduce all four types of vectors encoding the expression cassettes (i) to (iV) into cells.

Furthermore, it is preferable that the episomal vector used in the present invention contains a vector encoding the EBNA-1 gene sequence without encoding a reprogramming factor, p53DD, or the like, for example, pCXB-EBNA1.

As such a vector, Human iPS Cell Generation^{™} Episomal Vector Mix (manufactured by Takara Bio Inc.) can be preferably exemplified.

In the reprogramming step S2, it is preferable that the introduction of the reprogramming gene or the translation product thereof into the epithelial cells and/or the subsequent culture of the epithelial cells are performed in the presence of a ROCK inhibitor. The ROCK inhibitor to be used is not particularly limited as long as it can suppress the function of Rho kinase (ROCK), and examples thereof include Y-27632, Fasudil/HA1077, H-1152, Wf-536, and derivatives thereof, and an antisense nucleic acid against ROCK, an RNA interference inducing nucleic acid (For example, siRNA), a dominant negative variant, and expression vectors thereof.

In the present invention, one or more ROCK inhibitors may be used.

In the present invention, it is preferable to use Y-27632 as the ROCK inhibitor.

The concentration of Y-27632 to be used can be set in, for example, but is not limited to, a range of 100 nM to 50 µM, preferably 1 µM to 30 µM. In the present invention, the concentration of Y-27632 is preferably 10 µM.

In addition, it is preferable that the culture during the period from the introduction of the reprogramming gene or the translation product thereof into the epithelial cells in the reprogramming step S2 to the adherent culture step S3 is adherent culture on a culture surface coated with a coating agent.

The coating agent to be used is not particularly limited, and the method used in the culture step S1 can be adopted. In the present invention, it is preferable to use collagen I.

### (3) Adherent culture step S3

The adherent culture step S3 is a step of adhering and culturing the epithelial cells subjected to the reprogramming step S2 on a culture surface coated with an extracellular matrix.

In the adherent culture step S3, first, the epithelial cells are adhered to the inside of a culture vessel whose surface is coated with an extracellular matrix.

The extracellular matrix to be used is a laminin or a fragment thereof. Suitable examples of the laminin to be used include Laminin-α5β1γ1 (Laminin-511), Laminin-α5β2γ1 (Laminin-521), Laminin-α4β1γ1 (Laminin- 411), Laminin-α2β1γ1 (Laminin-211), and laminin fragments thereof, and it is more preferable to use Laminin-511 or Laminin-521. The laminin and the laminin fragment may also be recombinant.

It is preferable that the laminin or the laminin fragment has an integrin binding site.

It is more preferable that the laminin fragment forms a heterotrimer.

The type of integrin to which the laminin or the laminin fragment binds is preferably integrin α6β1, integrin α6β4, integrin α3β1, or integrin α7β1, and more preferably integrin α6β1. By using the laminin or the laminin fragment that binds to integrin α6β1, proliferation of iPS cells can be promoted.

As the laminin fragment having a binding activity to these integrins, a laminin fragment derived from at least one selected from Laminin-511, Laminin-521, Laminin-411 and Laminin-211 can be preferably exemplified, and the laminin fragment is more preferably a laminin fragment derived from Laminin-511 or Laminin-521, and still more preferably a laminin fragment derived from Laminin-511. Preferred examples of such a laminin fragment include an E8 fragment obtained by digesting the laminin with elastase and a recombinant of the E8 fragment.

In the present invention, it is more preferable to use Laminin-511E8. As Laminin-511E8, a commercially available product such as iMatrix-511 (manufactured by Nippi, Incorporated) can be used.

The culture vessel used in the adherent culture is not particularly limited as long as the cells can adhere to the culture vessel, and the same culture vessel as the culture vessel according to the culture step S1 can be adopted.

It is preferable that the adherent culture is performed using a serum-free culture medium after the first subculture. The serum-free culture medium preferably contains KSR or B27, or is under a xeno-free condition. Here, "xeno-free" means a condition in which any component derived from a biological species different from the biological species of the cells to be cultured is removed.

On the other hand, after the reprogramming step S2 and before the first subculture, it is preferable to culture the cells in a culture medium containing bovine pituitary extract (BPE), recombinant human insulin-like growth factor-I, hydrocortisone, bovine transferrin, and human epidermal growth factor.

When the epithelial cells obtained under serum-free and xeno-free conditions are used, it is preferable to culture them using a secondary culture medium after the reprogramming step S2 and before the first subculture.

It is preferable that the culture in the adherent culture step S3 is performed in the absence of feeder cells (feeder free). As the feeder-free culture medium to be used as an undifferentiated maintenance culture medium, a commercially available product can be used, and examples thereof include Essential 8 (manufactured by Life Technologies Corporation), S-medium (manufactured by DS Pharma Biomedical Co., Ltd.), StemPro (manufactured by Life Technologies Corporation), mTeSR1 (manufactured by STEMCELL Technologies), mTeSR2 (manufactured by STEMCELL Technologies), TeSR-E8 (manufactured by STEMCELL Technologies), and StemFit (manufactured by Ajinomoto co., inc.).

The absence of feeder cells (feeder free) means a condition substantially free of feeder cells (for example, a ratio of the number of feeder cells to the total number of cells is 3% or less).

In the adherent culture step S3, it is preferable to use Essential 8.

The Essential 8 medium contains L-ascorbic acid-2 phosphate magnesium, sodium selenium, insulin, NaHCO₃, transferrin, bFGF, and a TGFβ family signaling pathway agent (TGFβ1 or Nodal) as additives in DMEM/F12.

It is preferable to perform the adherent culture in the presence of a ROCK inhibitor. The ROCK inhibitor used in the adherent culture and a preferred form thereof are the same as those in the reprogramming step S2 described above.

Also in the adherent culture step S3, it is preferable to inhibit the function of p53 as in the reprogramming step S2. As a means for inhibiting the function of p53, the method described in the reprogramming step S2 can be appropriately adopted.

The period of the adherent culture is preferably 5 days or more, more preferably 10 days or more, and still more preferably 15 days or more.

By continuing the adherent culture, the appearance of cell colonies can be confirmed. Subsequently, it is preferable to select candidate colonies of iPS cells using existing methods.

Selection of candidate colonies of iPS cells can also be performed by visual morphology observation, confirmation of expression of iPS cell-specific surface antigens such as TRA1-60 and SSEA4, and the like.

In the reprogramming step S2 and the adherent culture step S3, in order to further enhance the establishment efficiency of iPS cells, a known substance having an action of improving the establishment efficiency may be used.

Examples of such substances include, but are not limited to, histone deacetylase (HDAC) inhibitors such as valproic acid (VPA), trichostatin A, sodium butyrate, MC 1293, and M344, DNA methyltransferase inhibitors such as 5'-azacitidine, G9a histone methyltransferase inhibitors such as BIX-01294, L-channel calcium agonists such as Bayk8644, p38 inhibitors, UTF1, Wnt signaling activators, 3'-phosphoinositide-dependent kinase-1 (PDK1) activators such as PS48, and the like.

The iPS cells produced according to the present invention can be used in various methods such as pharmaceutical raw materials and screening materials. For example, various cells such as muscle cells, blood cells, nerve cells, vascular endothelial cells, insulin secreting cells, and epithelial cells can be produced from the iPS cells using an existing differentiation inducing method. In addition, in vitro screening for drug efficacy or toxicity of a pharmaceutical candidate compound can also be performed using differentiated cells.

Furthermore, iPS cells are established using a sample of a subject (for example, hair), and by using a skin tissue or the like differentiated from the iPS cells unique to the subject, the effectiveness of pharmaceuticals, cosmetics, and the like according to each subject can be confirmed.

In addition, in one embodiment of the present invention, all of the culture step S1, the reprogramming step S2, and the adherent culture step S3 can be performed under serum-free and xeno-free conditions. That is, according to the present invention, a series of production steps can be performed under serum-free and xeno-free conditions.

### <Method for culturing epithelial cells>

The present invention also relates to a method for culturing epithelial cells, including culturing a sample, which contains an epithelial tissue, under serum-free and/or xeno-free conditions.

In the method for culturing epithelial cells of the present invention (hereinafter, referred to as culture method of the present invention), epithelial cells are obtained by culturing under serum-free and/or xeno-free conditions. The culture method of the present invention relates to the culture method under the serum-free and/or xeno-free conditions of the culture step S1 in <Method for producing induced pluripotent stem cells using epithelial cells> described above.

The "epithelial cells" cultured in the culture method of the present invention are a cell population obtained by culturing a sample containing an epithelial tissue, and include epithelial cells (for example, keratinocytes) expressing an epithelial cell marker and stem cells (for example, hair follicle stem cells (HFSC)) expressing an epithelial stem cell marker.

In the present invention, the epithelial cells may include keratinocytes, preferably hair follicle keratinocytes.

The culture method of the present invention is preferably a culture method for primary culture.

The primary culture in the present invention refers to culture after seeding a collected sample.

It is preferable that the epithelial tissue according to the present invention is a tissue including epithelial cells which are the object of the culture method of the present invention.

The sample containing the epithelial tissue used in the present invention is not particularly limited as long as it is obtained from the epithelial tissue, and for example, an epithelial tissue of a hair follicle may be used. In the present invention, it is preferable to use a tissue containing an outer root sheath as the sample containing the epithelial tissue. In addition, the sample containing the epithelial tissue used in the present invention may include cells present in the bulge region.

The sample containing the epithelial tissue may be collected from a mammal (for example, human and the like). In the culture method of the present invention, it is preferable to use a tissue collected from a human.

As a method for obtaining the tissue to be used, an existing tissue collection method can be adopted. When the sample containing the outer root sheath is collected, the collection can be performed by biopsy or by plucking hair. From the viewpoint of non-invasiveness, it is preferable to obtain the sample by plucking hair.

In the hair obtained by plucking, the outer root sheath is adhered to a portion above the hair bulb. In the present invention, it is preferable to culture the sample including the outer root sheath in a state of being adhered to the hair.

As a preferred embodiment (arbitrary antiseptic step, the culture according to the first step S11 to the third step S13, and the like) of the culture method of the present invention, the description of the culture step S1 in <Method for producing induced pluripotent stem cells using epithelial cells> described above is cited. In this case, the "tissue (sample) including the outer root sheath" can be appropriately replaced with the "sample including the epithelial tissue".

### Examples

Hereinafter, the present invention will be described in more detail with reference to examples, but the technical scope of the present invention is not limited to the following examples.

### <Example 1>

### (1) Reagents used, etc.

### (a) EpiLife medium

To a volume of 500 ml of EpiLife^{™} Medium with 60 µM calcium (Gibco), the total amount (5 mL) of Human Keratinocyte Growth Supplement (HKGS, Gibco), 5 mL of a penicillin-streptomycin mixed solution (Nacalai Tesque), and 500 µL of Amphotericin-B were added and mixed.

### (b) MEF-Conditioned medium

To 10 mL of EmbryoMax (registered trademark) MEF Conditioned Media (Merck), 0.1 mL of the penicillin-streptomycin mixed solution (Nacalai Tesque), 10 µL of Amphotericin-B and 0.1 mL of HKGS (Gibco) were added and mixed.

### (c) Antibiotic Mix

247 ml of HBSS was transferred to a sterile container, and 2.5 ml of the penicillin-streptomycin mixed solution (final concentration: 100 U/ml for penicillin, 100 µg/ml for streptomycin) and 250 µL of Amphotericin B solution (final concentration: 250 ng/ml) were added thereto.

### (d) Transformation solution

Human iPS Cell Generation^{™} Episomal Vector Mix (Takara Bio) and Opti-MEM (Thermo Fisher Scientific) were mixed, and FuGENE HD Transfection (Promega) was further added, then the mixture was left to stand at room temperature for 20 minutes. Immediately prior to transformation, an EpiLife medium was added.

### (e) Collagen coating

A collagen acidic solution I-PC (atelocollagen) of 5 mg/mL was diluted 10 times with 1 mM of hydrochloric acid, and the diluted collagen solution was added to 400 µL/well in the case of a 12-well plate for cell culture and 1000 µL/well in the case of a 6-well plate for cell culture, and the diluted collagen solution was left to stand at 37°C for 1 hour or more, and then removed by an aspirator. Then, the plate was washed three times with PBS before use.

### (f) iMatrix coating

4.5 µL of iMatrix-511 (Nippi) was diluted with 1 mL of DPBS (-/-), and the diluted solution was added to a 12-well plate for cell culture at 500 µL/well, and the diluted solution was left to stand at 37°C for 1 hour or at 4°C overnight. Thereafter, the diluted solution was removed and used.

### (2) Culture of epithelial cells

Five hairs of the subject (human) were plucked, and then immediately immersed in an Antibiotic Mix for a moment. Then, the portion including the outer root sheath of the hair was cut out by 1 mm to 2 mm in the Antibiotic Mix, and seeded 1 sample per well in 250 µL of a mixed culture medium (EpiLife + MEF-Conditioned medium + Antibiotic Mix) of a 12-well plate coated with a collagen solution (collagen acidic solution I-PC with a final concentration of 0.5 mg/mL (atelocollagen)).

1 day or 2 days after seeding, 250 µL of the mixed culture medium was replenished, and 3 days after seeding, 500 µL of the mixed culture medium was further replenished. Then, it was confirmed that the total liquid volume of the culture medium was about 0.75 mL 3 days after seeding, and stationary culture was performed until 6 days after seeding.

When the proliferated cells were not expanded by 1 mm to 2 mm or more in radius around the hair shaft 6 days after seeding, 250 µL of the mixed culture medium was removed, and 250 µL of the MEF-Conditioned medium was replenished. The MEF-conditioned medium was replaced until the cells had a radius of 1 mm to 2 mm or more.

Next, it was confirmed that the proliferated cells had expanded around the hair shaft by a radius of 1 mm to 2 mm or more, and then the culture medium was replaced. In the culture medium replacement, all the mixed culture medium was removed, and 500 µL of the EpiLife medium was added. When the cells have expanded around the hair shaft by a radius of 1 mm to 2 mm or more 6 days after seeding, the culture medium was replaced with the EpiLife medium without replacing the MEF-Conditioned medium.

2 days after the culture medium replacement, 500 µL of the EpiLife medium was replenished again, and 2 days after the culture medium replenishment, the culture medium was replaced with 500 µL of the EpiLife medium. Then, the replenishment and replacement of the culture medium were similarly continued at a frequency of once every 2 days.

In the developed region of the cells proliferated from the outer root sheath, the EpiLife medium was removed and the region was washed with DPBS prior to overpopulation of the cells, specifically when the cells were 50% to 80% confluent. Then, 0.5 mL of TrypLESelect (Gibco) was added, and the cells were left to stand in an incubator at 37°C. Thereafter, the cells were detached from the plate by pipetting, and the cells were collected in a tube of 15 mL. The recovered cells were centrifuged, and then the supernatant was removed and suspended in 1 mL of the MEF-Conditioned medium.

Then, the cells were seeded on a 6-well plate coated with a collagen solution (collagen acidic solution I-PC with a final concentration of 0.5 mg/mL (atelocollagen)) at 50,000/well, and cultured in the incubator at 37°C. The culture medium replacement with 500 µL of the EpiLife medium was continued until the cells were 50% to 80% confluent. At a stage when the cells were 50% to 80% confluent, iPS cells were induced.

### (3) Induction of iPS cells

First, the EpiLife medium was replaced with a transformation solution, and the cells were left to stand in an incubator at 37°C for 4 hours. Thereafter, the culture medium was replaced with 2 mL of the EpiLife medium containing 10 µM of Y27632 (the EpiLife medium containing Y27632), and the cells were cultured.

On the next day, the culture medium was removed and the cells were washed with DPBS (-/-), then 1 mL of TrypLESelect was added, and the cells were left to stand in an incubator at 37°C, and then were detached from the plate by pipetting. Further, 2 mL of a trypsin neutralizing solution (KURABO) was added, then 3 mL of the EpiLife medium containing Y27632 was added, and the cells were collected in a tube of 15 mL. The recovered cells were centrifuged, and then the supernatant was removed and suspended in 1 mL of the EpiLife medium containing Y27632.

Next, the cells were seeded on a 12-well plate coated with iMatrix-511 (Nippi) at 10,000 cells/well, and cultured in an incubator at 37°C (subculture day 0). After 1 days to 3 days of subculture, an Essential 8^{™} Flex medium containing 10 µM of Y27632 (Essential 8 containing Y27632) was added daily. After 4 days of subculture, the entire amount of the culture medium was replaced with Essential 8 containing Y27632, and thereafter, the culture medium was replaced daily.

### (4) Immunohistochemical staining

After 1 day of subculture, cellular immunostaining of iPS cell marker genes (OCT4, SOX2, NANOG, and TRA1-81) was performed in the following procedure.

First, the cells were washed with PBS and fixed with 4% paraformaldehyde. PBS containing 0.5% TritonX-100 was added and left to stand for 10 minutes for permeation processing, and blocking was further performed for 30 minutes with PBS containing 10% goat serum.

Subsequently, the cells were washed with PBS, then primary antibodies were added, and the mixture was left to stand at 4°C overnight. After washing again with PBS, a fluorescent-labeled secondary antibody (Alexa647 or Alexa488) and Dapi were added, and the mixture was left to stand for 30 minutes. After washing with PBS, a fluorescence image was obtained by a fluorescence microscope.

### (5) Results

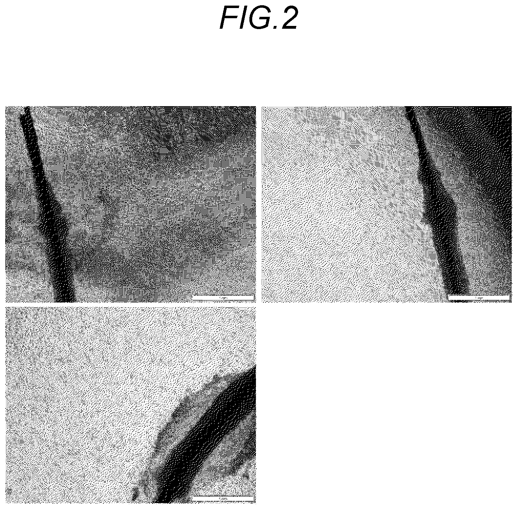

Fig. 2 shows the state of the epithelial cells expanded from the outer root sheath 12 days after seeding of the 12-well plate in (2) Culture of epithelial cells. As shown in Fig. 2, it was confirmed that many epithelial cells were proliferated from the periphery of the outer root sheath.

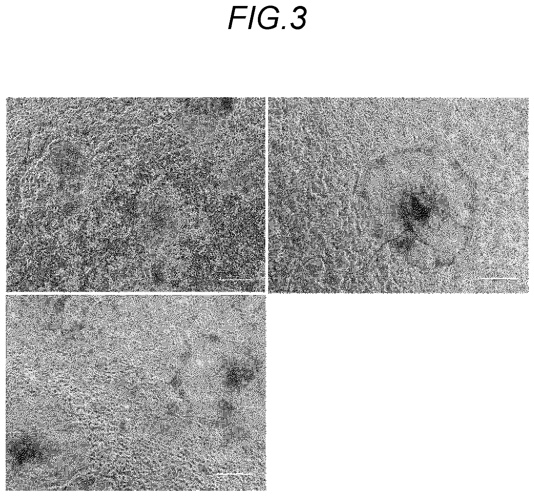

Fig. 3 shows the appearance of cells after 20 days of subculture in (3) Induction of iPS cells.

As shown in Fig. 3, a large number of colonies of iPS cells were confirmed by performing the production method of Example 1.

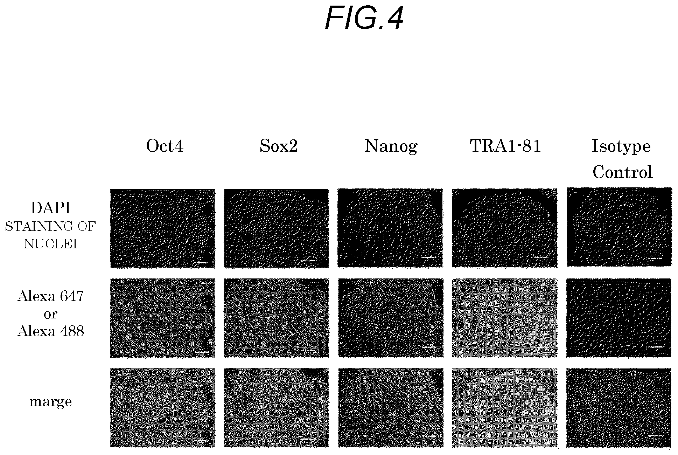

Furthermore, Fig. 4 shows that the proliferated cells expressed iPS cell markers and iPS cells having acquired pluripotency could be produced.

### <Comparative Example 1>

iPS cells were induced in the same procedure as in Example 1, except that the extracellular matrix used was changed to vitronectin in (3) Induction of iPS cells of Example 1.

As a result, in Comparative Example 1 using a plate coated with vitronectin, unlike Example 1 using the plate coated with iMatrix-511, apoptosis was induced, and after 1 week of subculture, almost all cells were dead.

### <Comparative Example 2>

In (3) Induction of iPS cells of Example 1, the episomal vector to be used was changed to Episomal iPSC Reprogramming Vectors (manufactured by Invitrogen Corporation) having no p53 inhibitory activity. At that time, iPS cells were induced in the same procedure as in Example 1 except for the vector to be used, and the number of colonies formed of iPS cells was confirmed.

As a result, it was found that the colony formation rate of iPS cells in Example 1 was remarkably excellent as compared with Comparative Example 2.

### <Consideration>

In view of the results of Example 1 and Comparative Examples 1 to 2, it has become clear that colonies of iPS cells can be efficiently obtained by using Example 1, which is the production method of the present invention. Specifically, in view of the results of Comparative Example 1, it has become clear that since iPS cells are subjected to the adherent culture on an adhesion surface coated with a laminin fragment such as iMatrix, apoptosis of the cells is suppressed and proliferation of the iPS cells is promoted.

In addition, the results of Comparative Example 2 show that inhibition of the function of p53 at the time of introduction of the reprogramming factor promoted the formation of iPS cell colonies. That is, it can be said that the formation of iPS cell colonies can be promoted and the production efficiency of iPS cells can be improved by simultaneously inhibiting the function of p53 at the time of expression of the reprogramming factor.

### <Example 2>

### (1) Reagents used, etc.

Reagents overlapping with those in Example 1 will be omitted as appropriate.

### (a) Mixed culture medium A (primary culture medium)

To a culture medium containing 90% of the Essential 8^{™} Flex medium (Gibco and Supplement added) and 10% of CTS Knockout SR XenoFree (Gibco), a penicillin-streptomycin mixed solution (added to a final concentration of 100 U/ml for penicillin and a final concentration of 100 ug/ml for streptomycin, Nacalai Tesque), Supplement S7 (Gibco, the final concentration was diluted 100 times from the original solution), EGF (Epidermal growth factor (EGF), human, FUJIFILM Wako Pure Chemical Corporation) at 20 ng/ml, IGF-I (insulin-like growth factor 1) at 20 ng/ml, hydrocortisone at 200 ng/ml, and transferrin at 10 ug/ml were added.

### (b) Mixed culture medium B (secondary culture medium)

The Essential 8^{™} Flex medium of the mixed culture medium A was replaced with EpiLife^{™} Medium, with 60 µM of calcium (Gibco), and the mixed culture medium B was produced in the same manner as the mixed culture medium A except for the above conditions.

### (2) Culture of epithelial cells

Hair was obtained by plucking from a subject (human) in the same procedure as in Example 1, and was disinfected with Antibiotic Mix. Then, 1 sample was seeded per well in 250 µL of the mixed culture medium A of a 12-well plate coated with collagen.

1 day or 2 days after seeding, 250 µL of the mixed culture medium A was replenished, and 3 days after seeding, 250 µL of the mixed culture medium A was further replenished. Then, it was confirmed that the total liquid volume of the culture medium was about 0.75 mL 3 days after seeding, and stationary culture was performed until 6 days after seeding.

6 days after seeding, 250 µL of the culture medium was removed and newly replaced with 250 µL of the mixed culture medium A. The culture medium was similarly replaced with the mixed culture medium A every other day until the cells expanded around the hair shaft by a radius of 1 mm to 2 mm or more. The culture using the mixed culture medium A was performed for 2 weeks to 3 weeks.

Next, it was confirmed that the proliferated cells had expanded around the hair shaft by a radius of 1 mm to 2 mm or more, and then the culture medium was replaced with the mixed culture medium B. In the replacement of the mixed culture medium B, all the mixed culture medium A was removed, and 500 µL of the mixed culture medium B was added.

2 days after the culture medium replacement, the entire amount of the culture medium was replaced with 500 µL of the mixed culture medium B. Thereafter, the replacement of the entire amount of the culture medium was similarly continued at a frequency of once every 2 days.

After culturing in the mixed culture medium B for 1 week to 2 weeks, in the developed region of the cells proliferated from the outer root sheath, the culture medium was removed and the region was washed with DPBS prior to overpopulation of the cells, specifically when the cells were 50% to 80% confluent. Then, 0.5 mL of TrypLESelect (Gibco) was added, and the cells were left to stand in an incubator at 37°C. Thereafter, the cells were detached from the plate by pipetting, and the cells were collected in a tube of 15 mL. The recovered cells were centrifuged, and then the supernatant was removed and suspended in 1 mL of the mixed culture medium B.

Next, the cells were seeded on a 6-well plate coated with a collagen solution at 50,000/well, and cultured in an incubator at 37°C. The culture medium replacement was performed with 500 µL of the mixed culture medium B, and the replacement was continued until the cells were 50% to 80% confluent. At a stage when the cells were 50% to 80% confluent, iPS cells were induced.

### (3) Induction of iPS cells

iPS cells were induced in the same procedure as in Example 1, except that the EpiLife medium containing Y27632 used in the culture after transformation was changed to the mixed culture medium B containing 10µM of Y27632. When the obtained cells were subjected to immunohistochemical staining in the same manner as in Example 1, it was confirmed that iPS cell markers were expressed. In addition, the tridermic differentiation potency of the obtained cells was confirmed.

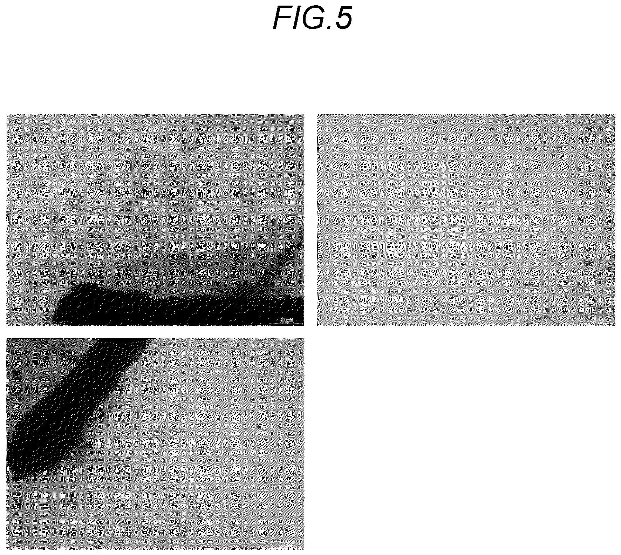

In view of the results of Example 2, it was shown that epithelial cells can be obtained from the tissue obtained by hair plucking under serum-free and xeno-free conditions (see Fig. 5). Using the epithelial cells, iPS cells could be induced under serum-free and xeno-free conditions. That is, according to the present invention, iPS cells can be produced using a tissue obtained by hair plucking under serum-free and xeno-free conditions.

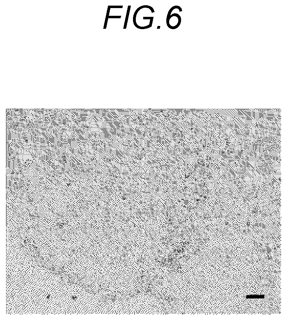

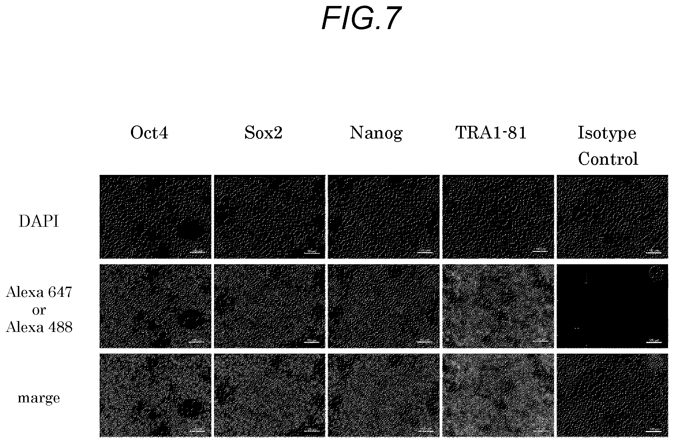

Fig. 6 shows the state of the iPS cells obtained in Example 2, and Fig. 7 shows the result of cellular immunostaining of iPS cell marker genes (OCT4, SOX2, NANOG, and TRA1-81) in the iPS cells of Fig. 6. Thus, it was confirmed that the proliferated iPS cells expressed a plurality of iPS cell markers and had pluripotency.

### [Example 3] Expression of markers of epithelial cells

Expression of epithelial cell markers was confirmed for the epithelial cells obtained in (2) Culture of epithelial cells of Example 1. The epithelial cell markers were assayed using the cells 7 days after seeding of the 12-well plate in the same procedure as in (4) Immunohistochemical staining, with Keratin-14 (KRT14) and CD200 as primary antibodies and Alexa647 or Alexa488 as secondary antibodies.

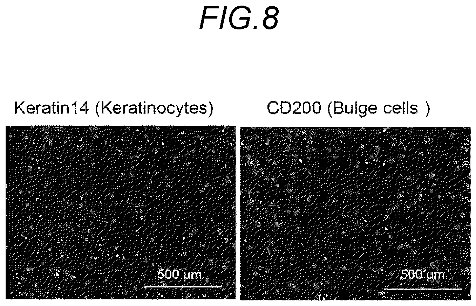

As a result, it was confirmed that the obtained cells were epithelial cells expressing both KRT14 and CD200 (Fig. 8).

### Industrial Applicability

The present invention can be applied to efficiency improvement of a method for inducing iPS cells, development of a pharmaceutical preparation using iPS cells, and the like.

### Reference Signs List

- S1: Culture step
- S11: First step
- S12: Second step
- S13: Third step
- S2: Reprogramming step
- S3: Adherent culture step

## Claims

1. A method for producing induced pluripotent stem cells using epithelial cells, the method comprising:
a reprogramming step including introducing a reprogramming gene for reprogramming a nucleus, or a translation product thereof, into the epithelial cells; and
an adherent culture step of adhering and culturing cells that have undergone the reprogramming step on a culture surface coated with a laminin or a fragment thereof.

2. The method according to claim 1, wherein the epithelial cells are derived from an outer root sheath.

3. The method according to claim 1, wherein the epithelial cells are derived from a tissue obtained by hair plucking.

4. The method according to claim 1, further comprising a culture step of culturing the epithelial cells used in the reprogramming step, wherein
the culture step includes a step of culturing a tissue including an outer root sheath to proliferate the epithelial cells.

5. The method according to claim 4, wherein the tissue including the outer root sheath is cultured in a state of being adhered to a hair.

6. The method according to claim 4, wherein the tissue including the outer root sheath is obtained from a hair obtained by plucking.

7. The method according to claim 1, wherein the reprogramming step includes a functional inhibition of p53.

8. The method according to claim 7, wherein the functional inhibition of p53 is induced by one or more substances selected from (a) to (c) below:
(a) a chemical inhibitor of p53;
(b) a dominant negative mutant of p53 and a nucleic acid encoding the dominant negative mutant of p53; and
(c) a siRNA and a shRNA against p53 and a DNA encoding the siRNA and the shRNA.

9. The method according to claim 1, wherein the method includes culturing cells, into which the reprogramming gene or a translation product thereof has been introduced during the reprogramming step, in a culture medium containing a ROCK inhibitor.

10. The method according to claim 4, wherein the culture step includes a step of bringing the tissue including the outer root sheath, which has been obtained, into contact with an antiseptic solution for less than 1 minute before culturing.

11. The method according to claim 4, wherein the culture step includes:
a step of seeding the tissue including the outer root sheath, which has been obtained, onto a plate coated with an extracellular matrix; and
a step of culturing the tissue including the outer root sheath seeded on the plate for 1 day to 3 days while replenishing a fresh culture medium without replacing an entire amount of a culture medium, and then subjecting the tissue including the outer root sheath to stationary culture for 2 days to 7 days without replacing or replenishing the culture medium to proliferate the epithelial cells.

12. The method according to claim 1, wherein the laminin is Laminin-511.

13. The method according to claim 1, wherein the reprogramming gene includes an Oct3/4 gene, a Klf4 gene, and a Sox2 gene.

14. The method according to claim 13, wherein the reprogramming gene includes a c-Myc gene or an L-Myc gene.

15. The method according to any one of claims 1 to 14, wherein the epithelial cells are keratinocytes.

16. The method according to claim 4, wherein a culture medium used in the culture step is serum-free and/or xeno-free.

17. The method according to claim 16, wherein the culture step includes a step of culturing in a stem cell culture medium.

18. The method according to claim 17, wherein the culture step includes a step of culturing in an epithelial cell culture medium after culturing in the stem cell culture medium.

19. The method according to claim 16, wherein the culture step includes: a step of culturing in a high calcium culture medium having a calcium concentration of 0.2 mM to 4.0 mM; and
a step of culturing in a low calcium culture medium having a calcium concentration of 40 µM to 80 µM after culturing in the high calcium culture medium.

20. The method according to any one of claims 17 to 19, wherein the stem cell culture medium and/or the high calcium culture medium contains epidermal growth factor (EGF), insulin-like growth factor-I (IGF-I), hydrocortisone, transferrin, basic fibroblast growth factor (bFGF), TGFβ1 (transforming growth factor-β1), L-ascorbic acid, selenium and insulin.

21. The method according to claim 18 or 19, wherein the epithelial cell culture medium and/or the low calcium culture medium contains epidermal growth factor (EGF), insulin-like growth factor-I (IGF-I), hydrocortisone, and transferrin.

22. The method according to any one of claims 4 to 14 and 16 to 19, wherein the method does not include a degradation step of degrading a component of an extracellular matrix in a tissue including the outer root sheath before seeding the tissue including the outer root sheath.

23. The method according to claim 22, wherein the degradation step is a step of processing the tissue with one or more selected from trypsin, collagenase, and dispase.
